# EUROPEAN PATENT APPLICATION

(11) **EP 1 637 587 A1**
(43) Date of publication of application: **22.03.2006**
(21) Application number: 04730671.7
(22) Date of filing: 30.04.2004
(51) Int. Cl.: C12M 1/34

(54) **MICRO CHAMBER FOR CELL CULTURE**

(30) Priority: 19.05.2003 JP 2003139773
(71) Applicant: Japan Science and Technology Agency, Kawaguchi-shi, Saitama 332-0012 (JP)
(72) Inventor: YASUDA, Kenji, Koutou-ku, Tokyo 135-0052 (JP); TAKAHASHI, Kazunori, Mitaka-shi, Tokyo 181-0004 (JP)
(74) Representative: Wilson Gunn
(86) International application number: PCT/JP2004/006283
(87) International publication number: WO 2004/101734

(57) **Abstract**

A new micro-chamber is provided wherein a channel capable of selectively recovering a migrating cell in the micro-chamber can be opened or closed by reversibly changing the shape of the micro-chamber during culture.

The micro-chamber comprises a cell culture section 110 formed in an elastic polymer which is optically transparent in visible regions, channels 108, 109 positioned at both ends of the cell culture section, air reservoir 105, 106 for controlling, by expansion or contraction, the open or close state of the channels, air passages 103, 104 for applying pressurization or depressurization to the air reservoirs, and connecting joints 101, 102 to an air pressure control section, all of which are disposed on an optically transparent base plate 112 such as a slide glass etc., whereby a culture solution containing cells can be continuously passed to a direction of flow 107.

## Description

### Field of the Invention

The present invention relates to a micro-chamber for the culture of cells, more particularly, it relates to a cell culture micro-chamber by which cells can be cultured in one cell unit while observing the state of a cell with a microscope.

### Prior Art

Up to date, for the observation of a change in state of cells and a response to chemical agents, etc. of cells, it is common to observe the average value of some values of a cell population on the assumption that it is a property of one cell. However, actually, it is seldom that cells synchronize in their cell cycles in a group of them. Thus, each cell develops a protein in a different cycle.

In order to solve these problems, a technique such as a synchronous culture process, etc. has been developed. However, because the cultured cell is not derived from one completely identical cell, there was a possibility that a difference in development of a protein is caused by a difference in gene of each cell derived before culture. Actually, when analyzing the results of responses to irritation, it was difficult to clarify whether its fluctuation derives from that of general responses possessed by a cell' reaction mechanism itself or whether it is derived from a difference in cell (that is, a difference in gene information).

For the same reasons, with respect to cell lines it was difficult to clarify whether the reproducibility of responses to irritation fluctuates due to a difference in gene of each cell because it is generally not cultured from one completely identical cell.

Further, from the fact that there are two types of irritation (signal) to cells, i.e., one being given by the amounts of signal substance, nutriment and dissolved gas contained in a solution in the circumference of a cell, and the other being provided by the physical contact between cells, it was the circumstance that it is difficult to judge its fluctuation.

On the other hand, heretofore, when cells are to be observed in a study field of biotechnology, it is common either to observe them by removing a portion of a cell group cultured in a large culture vessel and setting it on a microscope, or to conduct the observation with a microscope by enclosing the entire microscope with a plastic container to control the temperature and then placing another small container within the enclosure under the control of carbon dioxide concentration and humidity. Then, it is designed to exchange the used culture solution with a new culture solution while culturing cells whereby the solution conditions are maintained constant.

For example, there is a process of maintaining nutrient conditions constant by means of a mechanism wherein a circulating pump operates upward and downward the level of a culture medium relative to the surface of a base material between a level higher than the upper end edge of the base material and a level lower than the lower end edge thereof in such a manner that when it decreases to the lower level, a culture medium is fed, while when it increases to the higher level, a culture medium is discharged (Japanese Patent Application Public Disclosure (Kokai) Hei 10 - 191961).

Further, there is a process of maintaining the nutrient conditions of a culture vessel constant by inserting in a culture vessel one end of each of an inlet tube for introducing a new culture medium into the culture vessel, an outlet tube for discharging a culture medium from the culture vessel and a gas tube for communicating a gas portion of the culture vessel with a pump, wherein the inlet tube, the outlet tube and the gas tube are provided on their respective conduit line with a filter for preventing the intrusion of bacilli into the culture vessel (Japanese Patent Application Public Disclosure (Kokai) Hei 8 - 172956).

However, in either of these inventions, it was impossible to culture cells are cultured while controlling the solution environment of a cell to be cultured and the physical contact between cells.

Accordingly, the inventors solved these problems, and invented a technique of selecting a specific new one cell only and culturing the one cell as a cell line, a technique wherein, when observing cells, the solution environment conditions of the cells are controlled and the cell concentration in the vessel is maintained constant, and a technique of observing the culture while specifying interacting cells (Japanese Patent Application Public Disclosure (Kokai) 2002 - 153260).

### Problems to be solved by the Invention

However, referring to the photo pincette technique used in the above Patent Application (Japanese Patent Application Public Disclosure (Kokai) 2002 - 153260), the latitude of its capturing power is on the extent of a piconewton, which was sufficient to capture a floating cell but insufficient to capture a cell migrating for itself. Further, it was difficult to recover selectively a migrating cell during culture from a culture section.

Accordingly, the inventors have made various studies on the above micro-chambers, and found a new micro-chamber wherein a channel capable of selectively recovering a migrating cell in the micro-chamber can be opened or closed by reversibly changing the shape of the micro-chamber during culture.

### Means to solve the Problems

That is, the present invention is a cell culture micro-chamber comprising a cell culture section, at least two channels for connecting the cell culture section to the outside, a means for opening or closing the channels, and a means for optically observing the cell culture section and the opening or closing of the channels, wherein one of the channels is a flow path through which a culture solution which may contain cells can be injected into the cell culture section, while another one of the channels is a flow path through which a culture solution which may contain cells can be discharged from the cell culture section, at least a portion of said channels is surrounded by an elastic material, and the means for opening and closing is for opening or closing the channels or altering the width of the channels by pressing or drawing the channels from outside in a direction substantially perpendicular to the observation direction of the means for optically observing.

Among optical observation means used herein includes optical microscopes, video recorder apparatus, cameras etc. They can be connected with a personal computer etc. to conduct a picture treatment. For easy observation, they can be also used with a light radiation apparatus.

It is preferable that the width of a channel when not operating the means for opening and closing is on the same extent as the size of a target cell. Accordingly, a suitable channel width may vary with the size of a target cell. In case that the width of a channel when not operating the means for opening and closing is somewhat narrower than the size of a target cell, the cell does not pass through the channel in a normal state, and passes through the channel only when it is opened. Therefore, this structure is suitable to separate cells.

The means for opening and closing can be one that opens or closes a channel or alters its width by applying an external force to the channel. They may be any of a means utilizing a mechanical force, a means provided with a space wherein the volume is changed, and the like.

It is preferable that the means for opening and closing has a space adjacent to the channels, the space being filled with a gas or liquid and the size of the space being altered by changing the pressure of the gas or liquid, whereby the channels are opened or closed, or their widths are altered. It is most convenient that this space is filled with air so that the resulting air-filled space is used as an air reservoir whereby the opening or closing of the channel is controlled by its air pressure.

The channels may be surrounded over its entire by an elastic material, or only its means for opening and closing side may be made of an elastic material. It is preferable that the surroundings of the space and the channels are formed from the same material, and they are disposed such that the change in size of the space has a direct effect on the width of the channel.

The elastic material may be any elastic material. It is convenient to use a synthetic polymer having no adverse effect on the culture of cells. Particularly, it is preferable that the elastic material is a silicone-type resin.

For optically observing the cell culture section and the opening or closing of a channel in a cell culture micro-chamber, it is preferred to fabricate only necessary portions thereof with a transparent material. The entire may be formed with a transparent elastic material.

### Brief Description of the Drawing

Fig. 1 is a schematic view illustrating one example of basic constituents of the present invention.
Fig. 2 is a schematic view illustrating one example of the opening or closing process of channels.
Fig. 3 is a microphotograph showing one example of cell culture micro-chamber processing processes.
Fig. 4 is a microphotograph showing one example of molds and polymer cell culture micro-chambers used in the cell culture micro-chamber processing process of the present invention.
Fig. 5 is a microphotograph showing the open and close states of the channel of the cell culture micro-chamber.
Fig. 6 is a microphotograph showing a case where a cell passes through the opened channel of a cell culture micro-chamber, in which the arrow indicates a cell.

In these drawings, reference numerals indicate those parts as follows;
101, 102 : a connecting joint to an air pressure control section
103, 104: a passage of air
105, 106, 202 : an air reservoir
107 : a flow of a solution (a culture solution containing a cell)
108, 109, 111, 201, 204: a channel
110 : a cell culture section
112, 302, 304 : a glass base plate
113, 303 : a polymer (a silicone-type resin)
203 : a cell
301 : a mold
305 : a connector to an air pressure control section

### Embodiments of the Invention

The following illustrates the details of the cell culture micro-chamber of the present invention. However, the present invention is not to be restricted in any way to this cell culture micro-chamber.

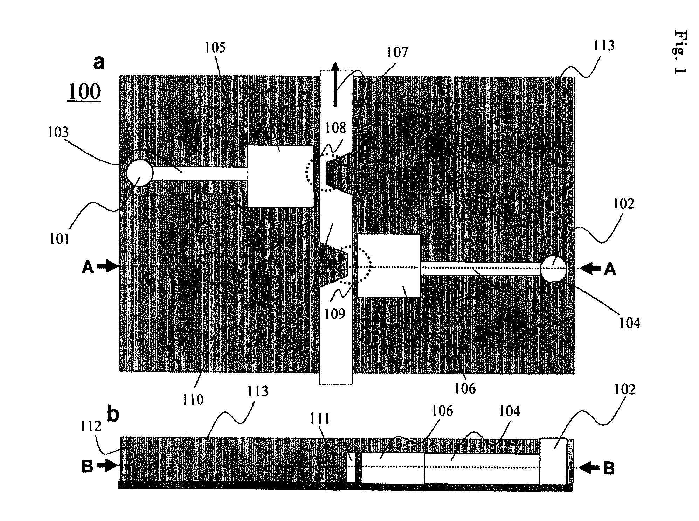

Fig.1 shows one example of basic constitutions of cell culture micro-chambers of the present invention. As illustrated in (B-B) horizontal section view of Fig.1a and (A-A) longitudinal section view of Fig.1b, the cell culture micro-chamber 100 of the present invention comprises a cell culture section 110 formed in an elastic polymer which is optically transparent in visible regions, air reservoirs 105, 106 for controlling, by expansion or retraction, the open or close state of channels 108, 109, 111 positioned in both ends of the cell culture section, air passageways 103, 104 for pressurizing or depressurizing the air reservoirs, and connecting joints 101, 102 to an air control section, all of which are disposed on an optically transparent base plate 112 such as a slide glass etc., whereby a solution (a culture solution containing cells) can be continuously passed to a direction of flow 107.

As shown in Fig.1, it is possible to capture or discharge a culturing cell from a culture section 110 depending on the open or close state of two channels 108, 109. Here, by the closing or opening of the channel is meant that the width of a channel 111 is adjusted by controlling the pressure of air in an air reservoir to expand or retract the wall surface of a polymer surrounding the channel. The channel is horizontally disposed on a plane surface perpendicular to the light axis of an optical microscope so that the width of a channel can be determined by means of an optical instrument means. Thus, by the use of, for example, an optical microscope, it is possible to confirm the width, i.e., the open or close state, of a channel by a mere observation without necessity for flowing a sample. Accordingly, under the visual confirmation of this open state, it is possible to control the air pressure of air reservoirs 105, 106. Further, by the automatic measurement of this open state by means of a picture treatment, it is also possible to do a feed back control for a predetermined open state.

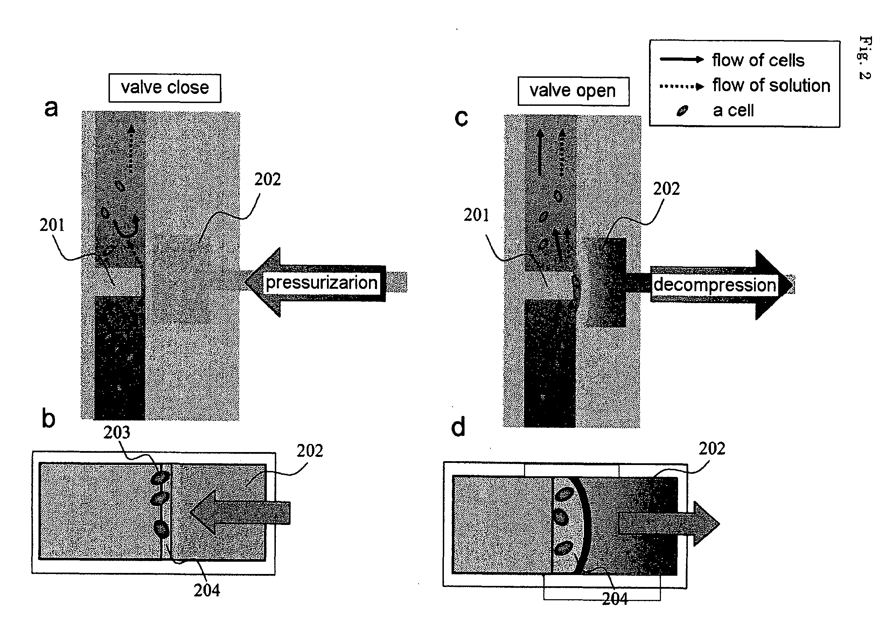

Next, the opening or closing of a channel will be described with reference to Fig.2. Fig. 2a and b are, respectively, horizontal section and cross section views showing a case where the channel is moderately closed so that only a solution passes through a channel but a cell 203 does not. By introducing air into an air reservoir 202 for pressurization, a channel 204 is closed so that the size of a cell passing therethrough can be controlled according to the extent of its pressurization.

On the other hand, Fig.2c and d are, respectively, horizontal section and cross section views showing a case where the channel is opened so that not only a solution but also a cell passes through a channel 201. By discharging air from an air reservoir 202, a channel 204 is opened so that all of cells can be passed therethrough according to the extent of its depressurization.

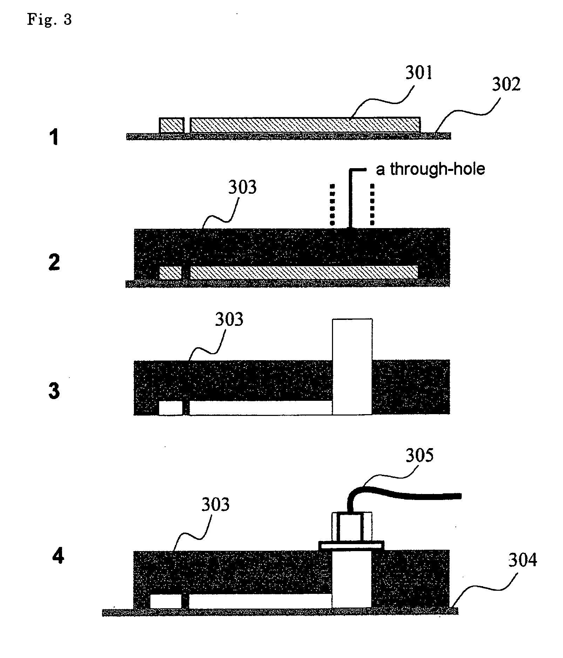

Fig.3 illustrates one example of a process for making cell culture micro-chambers. First, a mold is formed according to a fine processing technique such as photolithography etc. (Fig. 3-1). For example, a photo curable, thick, resist material SU-8 can be optically cured on a glass plate 302 to form a mold 301 thereon. Then, an optically transparent elastic polymer can be poured and cured on the mold to transfer the shape of the mold to the polymer (Fig.3-2). Here, as the elastic polymer material which is optically transparent in visible regions, for example, a silicone resin (a polydimethylsiloxane etc.) can be used. After the polymer was cured, a through-hole for flowing air or a solution is formed therein with the use of a perforating machine and then the polymer is peeled from the mold (Fig.3-2). Finally, this fine structure-transferred polymer is adhered on a glass base plate 304, which is further provided with a connector to an air pressure control section, and inlet and outlet sections for a solution. The final product thus formed can be used as a micro-chamber for cell culture.

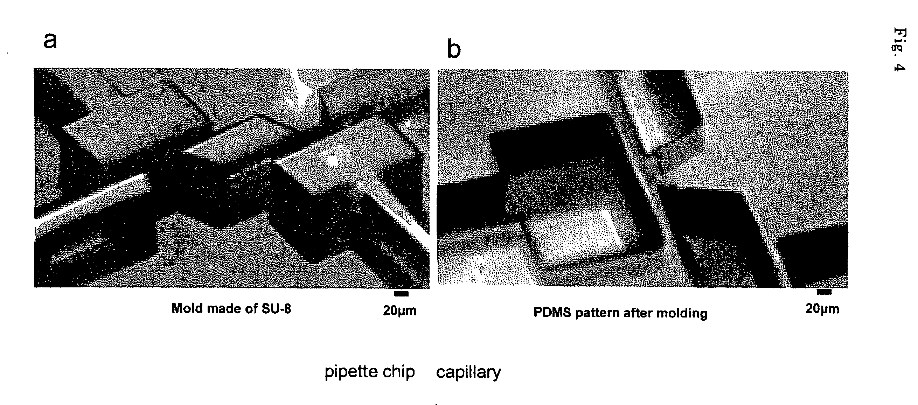

Fig.4 is an optical microphotograph showing one example each of a mold (Fig.4a) for a cell culture micro-chamber made using a photo-curable resin SU-8 according to the process of Fig.3 and of a polymer fine structure (Fig.4b) formed by the transfer of the mold. From this optical microphotograph, it is seen that the fine structure of a mold can be precisely transferred to a polymer.

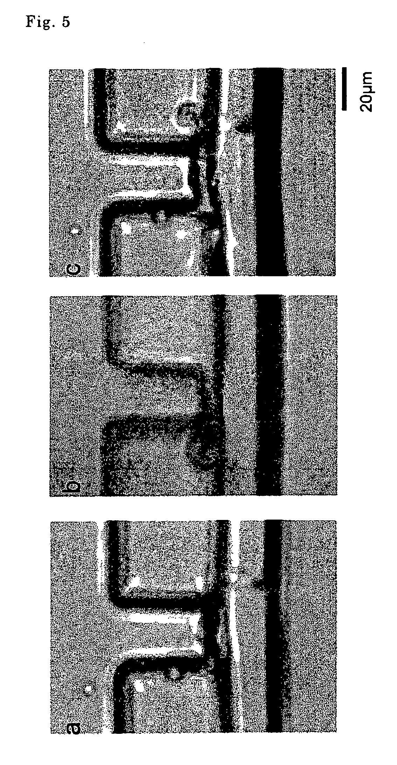

Fig.5 is a series of microphotographs showing the functions of a channel in a cell culture micro-chamber of the present invention. Fig.5a shows a case where a channel is closed by pressurizing an air reservoir with air so that neither a cell nor a solution passes therethrough. Fig.5b shows a case where, by the suction of an air reservoir with a negative pressure, a channel is opened to the extent that a cell does not pass therethrough. As seen from this microphotograph, the cell is drawn up to the channel by a flow of the solution but can not pass therethrough. The open amount of the channel can be visually confirmed by means of an optical microscope. Thus, the open value of the channel can be controlled by a mere visual observation without necessity for flowing a cell. Fig.5c shows a case where, by further opening the channel, a cell is flowed.

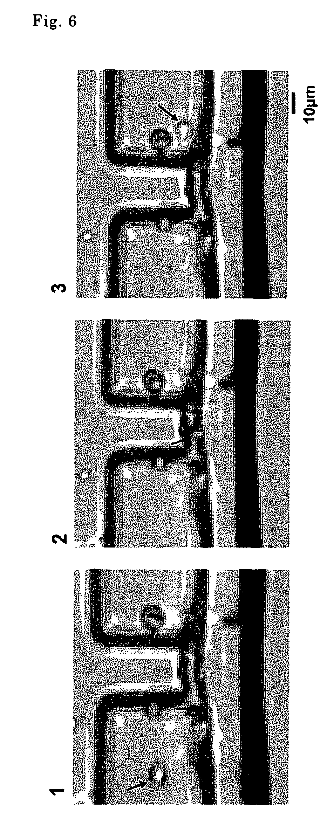

Fig.6 is a microphotograph showing a process wherein a cell passes through a channel. The cell (the arrow) in the left side of a channel (Fig. 6-1) passes through the channel (Fig.6-2) to transfer to the right side of the channel (Fig.6-3).

### Advantages of the Invention

The cell culture micro-chambers of the present invention have the following characteristic features:
The cell culture section is connected with the outside through a channel only. Therefore, cells are not in contact with an opening and closing apparatus etc. so that an extra load is not imposed on the cells.
Because the means for opening and closing is in a direction substantially vertical to the observation direction of opening or closing of a channel, the observation is not affected by the opening or closing of the channel. Further, simultaneously with the observation of a cell passing or not passing through a channel, the open or close state of the channel can be observed.
Due to these characteristic features, it is possible to separate a single cell from a cell culture solution.
Thus, it is possible to culture migrating cells etc. while controlling the number of the cells in a container, which was considered impossible up to date. Further, it is possible to recover selectively cells in the inside of a container.

## Claims

1. A cell culture micro-chamber comprising a cell culture section, at least two channels for connecting the cell culture section to the outside, a means for opening or closing the channels, and a means for optically observing the cell culture section and the opening or closing of the channels, wherein one of the channels is a flow path through which a culture solution which may contain cells can be injected into the cell culture section, while another one of the channels is a flow path through which a culture solution which may contain cells can be discharged from the cell culture section, at least a portion of said channels is surrounded by an elastic material, and the means for opening and closing is for opening or closing the channels or altering the width of the channels by pressing or drawing the channels from outside in a direction substantially perpendicular to the observation direction of the means for optically observing.

2. A cell culture micro-chamber according to claim 1 wherein the width of the channels when not operating the means for opening and closing is on the same extent as the size of a target cell.

3. A cell culture micro-chamber according to claim 1 or 2 wherein the means for opening and closing has a space adjacent to the channels, the space being filled with a gas or liquid and the size of the space being altered by changing the pressure of the gas or liquid, whereby the channels are opened or closed, or their widths are altered.

4. A cell culture micro-chamber according to any one of claims 1 to 3 wherein the elastic material is a silicone-type resin.
